# DEMANDE DE BREVET EUROPEEN

(11) **EP 1 060 756 A1**
(43) Date de publication de la demande: **20.12.2000**
(21) Numéro de dépôt: 00401507.9
(22) Date de dépôt: 29.05.2000
(51) Int. Cl.: A61M 16/00, A61B 5/09

(54) **Appareil de diagnostic ou de traitement des troubles respiratoires du sommeil et procédé de fonctionnement**

(30) Priorité: 14.06.1999 FR 9907489
(71) Demandeur: TAEMA, F-92182 Antony Cédex (FR)
(72) Inventeur: Vega , Enrique, 75005 Paris (FR)
(74) Mandataire: Le Moenner, Gabriel

(57) **Abrégé**

L'invention concerne un procédé de fonctionnement d'un appareil de diagnostic ou de traitement des troubles respiratoires du sommeil d'un utilisateur, ledit appareil comprenant une turbine motorisé permettant de délivrer un flux de gaz respiratoire sous pression variable ou constante dans un circuit patient relié audit utilisateur.

Ce procédé se compose des étapes de détermination d'une image du débit respiratoire du patient à partir des variations du courant électrique consommé par la turbine ; détermination d'une image du volume respiratoire du patient par intégration de l'image du débit respiratoire du patient ; détermination de la moyenne mobile de l'image du volume respiratoire du patient sur n cycles respiratoires ; et détermination d'une limitation de débit respiratoire, inférieure à celle d'une apnée ou d'une hypopnée, représentative d'un événement de type AER.

## Description

La présente invention se rapporte à un procédé de fonctionnement ou de commande d'un appareil de ventilation artificielle permettant de diagnostiquer ou de traiter les troubles du sommeil de type AER, pendant lesquelles un patient est soumis à des limitations de son volume respiratoire moins importantes que celles ayant lieu durant une période d'apnée ou d'hypopnée, mais néanmoins assez préoccupantes pour nécessiter un traitement, ainsi qu'un tel appareil.

Actuellement, de nombreux appareils de ventilation artificielle de patient permettent d'assurer une détection efficace de certains troubles respiratoires du sommeil, à savoir les apnées ou les hypopnées.

Une apnée est définie comme un trouble du sommeil se caractérisant chez une personne par un arrêt de l'activité respiratoire pendant une période de temps variable durant son sommeil, c'est-à-dire une réduction de plus de 90% de l'amplitude du débit respiratoire normal de cette personne.

Par ailleurs, une hypopnée est définie, quant à elle, comme un trouble du sommeil se caractérisant chez une personne par une diminution de 50% l'amplitude du débit respiratoire de cette personne pendant une période de temps variable durant son sommeil.

Il existe aujourd'hui de nombreuses méthodes et appareils permettant de diagnostiquer ou de traiter ces deux types de troubles respiratoires et, à ce titre, on peut citer notamment les documents US-A-5,245,995, US-A-5,148,802, US-A-5,199,424, US-A-4,655,213, US-A-5,335,654, US-A-5,353,788, US-A-5,458,137 et EP-A-505232.

Toutefois, des études récentes ont montré qu'il existait un troisième type d'événements respiratoires engendrant des troubles respiratoires du sommeil chez la personne qui en est sujette, à savoir les évènements respiratoires qui n'impliquent pas de réduction de 50% à quasi 100% du débit respiratoire, comme dans le cas d'une hypopnée ou d'une apnée, mais qui engendrent, par contre, une réduction de volume respiratoire pendant la durée de l'événement, suivie d'une reprise respiratoire et un micro-éveil de la personne à la fin de l'événement.

Durant la reprise respiratoire et le micro-éveil, on assiste à une augmentation importante du volume de gaz respiratoire inspiré par la personne.

De tels évènements respiratoires correspondent à des périodes de limitation de flux et sont appelés évènements de type AER, pour Autres Evénements Respiratoires, ce qui permet de les distinguer des apnées et des hypopnées.

Or, pouvoir assurer une détection efficace de ces périodes de limitation de flux correspondant à des évènements de type AER est primordial si l'on souhaite réaliser un diagnostic et/ou un traitement correct des troubles respiratoires du sommeil d'une personne.

De là, le problème qui se pose est de proposer un appareil capable de réaliser une détection efficace des périodes de limitation de flux correspondant à des événements de type AER chez une personne souffrant de troubles respiratoires du sommeil, de manière à ce que le médecin ou analogue puisse réaliser ensuite un diagnostic efficace de tels troubles AER et prescrire un traitement approprié.

La présente invention concerne alors un procédé de fonctionnement d'un appareil de diagnostic ou de traitement des troubles respiratoires du sommeil d'un utilisateur, ledit appareil comprenant au moins une turbine motorisé permettant de délivrer un flux de gaz respiratoire sous pression variable ou constante dans au moins un circuit patient susceptible d'être relié audit utilisateur, comprenant les étapes de :
(a) détermination d'une image du débit respiratoire (IdD) du patient à partir d'une détection et/ou d'un suivi des variations du courant électrique consommé par la turbine pendant au moins une partie d'au moins un cycle respiratoire donné ;
(b) détermination d'une image du volume respiratoire (IdV) du patient par intégration de l'image du débit respiratoire du patient (IdD) pour obtenir l'aire (Acc) du courant consommé durant au moins au moins une partie dudit au moins un cycle respiratoire donné ;
(c) détermination de la moyenne mobile (mm-ldV) de l'image du volume respiratoire du patient sur les n cycles respiratoires ayant précédé ledit au moins un cycle respiratoire donné, avec n > 5 ;
(d) détermination d'au moins une information représentative d'au moins une limitation de débit respiratoire durant au moins ledit cycle respiratoire donné :
   (i) par comparaison de l'image du volume respiratoire (IdV) déterminée à l'étape (b) avec la moyenne mobile (mm-ldV) de l'image du volume respiratoire déterminée à l'étape (c), et/ou
   (ii) par calcul du rapport entre l'image du volume respiratoire (IdV) déterminée à l'étape (b) et l'aire (Asp) d'un sinus pur préfixé et/ou l'aire (Asc) d'un signal carré préfixé ;
(e) mémorisation et/ou affichage et/ou impression d'au moins une information déterminée à l'étape (d).

Selon le cas, le procédé de l'invention peut comprendre l'une ou plusieurs des caractéristiques suivantes :
- à l'étape (a), on détermine une image du débit respiratoire (IdD) et/ou à l'étape (b), on détermine une image du volume respiratoire (IdV) sur au moins une partie d'au moins 2 cycles respiratoires donnés successifs, de préférence sur 3 ou 4 cycles respiratoires donnés successifs.
- à l'étape (c), la moyenne mobile (mm-ldV) de l'image du volume respiratoire du patient est déterminée sur les n cycles respiratoires ayant précédé ledit au moins un cycle respiratoire donné, avec n > 10, de préférence n est compris entre 15 et 40, préférentiellement encore entre 20 et 30.
- il comporte, en outre, au moins une étape de mémorisation et/ou affichage et/ou impression d'au moins une donnée choisie dans le groupe formé par l'image du débit respiratoire (IdD), l'image du volume respiratoire (IdV), et la moyenne mobile (mm-ldV) de l'image du volume respiratoire.
- à l'étape (d), on soustrait une valeur de moyenne mobile (mm-ldV) de l'image du volume respiratoire déterminée à l'étape (c) à la valeur de l'aire (Acc) du courant consommé correspondant à l'image du volume respiratoire (IdV) déterminée à l'étape (b).

En outre, l'invention concerne aussi un appareil de diagnostic ou de traitement des troubles respiratoires du sommeil d'un utilisateur susceptible de fonctionner selon un procédé de fonctionnement selon l'invention, ledit appareil comprenant un circuit de gaz susceptible d'être relié audit utilisateur et au moins une turbine motorisé permettant de délivrer un flux de gaz respiratoire sous pression variable ou constante dans au moins ledit circuit de gaz, comprenant :
- des moyens de détection et/ou de suivi des variations du courant électrique consommé par la turbine pendant au moins une partie d'au moins un cycle respiratoire donné ;
- des moyens de détermination d'une image du débit respiratoire (IdD) du patient ;
- des moyens de détermination d'une image du volume respiratoire (IdV) du patient représentée par l'aire (Acc) du courant consommé durant au moins au moins une partie dudit au moins un cycle respiratoire donné, comprenant des moyens intégration de l'image du débit respiratoire du patient (IdD) pour obtenir ladite l'aire (Acc) du courant consommé ;
- des moyens de détermination de la moyenne mobile (mm-ldV) de l'image du volume respiratoire du patient sur les n cycles respiratoires ayant précédé ledit au moins un cycle respiratoire donné, avec n > 5 ;
- des moyens de détermination d'au moins une information représentative d'au moins une limitation de débit respiratoire durant au moins ledit cycle respiratoire donné (i) par comparaison de l'image du volume respiratoire (IdV) déterminée à l'étape (b) avec la moyenne mobile (mm-ldV) de l'image du volume respiratoire déterminée à l'étape (c), et/ou (ii) par calcul du rapport entre l'image du volume respiratoire (IdV) déterminée à l'étape (b) et l'aire (Asp) d'un sinus pur préfixé et/ou l'aire (Asc) d'un signal carré préfixé ; et
- des moyens de mémorisation et/ou affichage et/ou impression d'au moins une information de débit respiratoire et/ou d'au moins une donnée choisie dans le groupe formé par l'image du débit respiratoire (IdD), l'image du volume respiratoire (IdV), et la moyenne mobile (mm-ldV) de l'image du volume respiratoire.

Selon le cas, l'appareil de l'invention peut comprendre l'une ou plusieurs des caractéristiques suivantes :
- les moyens de détection et/ou de suivi des variations du courant électrique consommé par la turbine permettent de déterminer une image du débit respiratoire (IdD) et/ou de déterminer une image du volume respiratoire (IdV) sur au moins une partie d'au moins 2 cycles respiratoires donnés successifs, de préférence sur 3 ou 4 cycles respiratoires donnés successifs.
- les moyens de détermination de la moyenne mobile permettent de déterminer la moyenne mobile (mm-ldV) de l'image du volume respiratoire du patient sur n cycles respiratoires ayant précédé au moins un cycle respiratoire donné, avec n > 10, de préférence n est compris entre 15 et 40, préférentiellement encore entre 20 et 30.
- les moyens de détermination d'au moins une information représentative d'au moins une limitation de débit respiratoire permettent de soustraire une valeur de moyenne mobile (mm-ldV) de l'image du volume respiratoire déterminée par les moyens de détermination de la moyenne mobile à la valeur de l'aire (Acc) du courant consommé correspondant à l'image du volume respiratoire (IdV) déterminée par les moyens de détermination d'une image du volume respiratoire (IdV).

La présente invention va maintenant être expliquée plus en détail.

De façon plus générale, les aléas de la respiration d'un patient en phase de sommeil peuvent parfois conduire à des diminutions ou limitations de débit respiratoire qui ne sont pas des troubles du type AER ou d'autres artéfacts susceptibles de conduire à des diagnostics erronés.

Pour y remédier, il peut être souhaitable de fixer des critères de détection et/ou discriminations de ce type d'artéfacts, par exemples une durée trop courte du temps inspiratoire ou expiratoire, une quantité trop faible de courant inspiratoire ou expiratoire, etc.

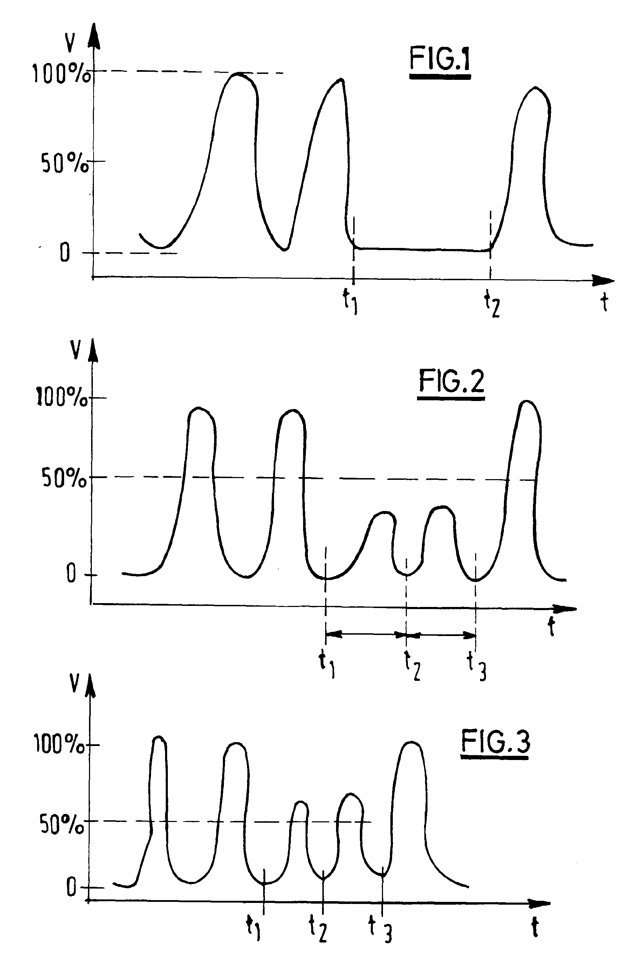

Les figures 1 à 3 données à titre illustratif mais non limitatif illustrent les 3 types de troubles du sommeil pouvant être observés chez un patient.

La figure 1 schématise un cycle respiratoire chez un patient sujet à des troubles de type apnées du sommeil. Comme on peut le voir, entre les temps t1 et t2, le volume respiratoire diminue considérablement par rapport au volume respiratoire normal (100 %) jusqu'à atteindre un volume respiratoire de l'ordre ou proche de 0%, c'est-à-dire des périodes durant lesquelles le patient ne respire pas car étant en période d'apnée.

Par ailleurs, la figure 2 schématise un cycle respiratoire chez un patient sujet à des troubles de type hypopnées du sommeil. Comme on peut le voir, entre les temps t1 et t2, le volume respiratoire diminue nettement par rapport au volume respiratoire normal (100 %) jusqu'à atteindre un volume respiratoire de l'ordre ou inférieur à 50% du volume normal, c'est-à-dire des périodes durant lesquelles le patient ne respire pas complètement car étant en période d'hypopnée. Toutefois, durant les périodes d'hypopnées, le volume respiratoire diminue beaucoup moins qu'en périodes d'apnées.

Enfin, la figure 3 schématise un cycle respiratoire chez un patient sujet à des troubles du sommeil de type AER. Comme on peut le voir, dans ce cas, entre les temps t1 et t2, le volume respiratoire diminue un peu par rapport au volume respiratoire normal (100 %) jusqu'à atteindre un volume respiratoire de toujours supérieur à 50% du volume normal, c'est-à-dire que durant ces périodes de limitation de la respiration, le volume respiratoire diminue moins qu'en périodes d'hypopnées rendant alors ces troubles de type AER plus difficiles à détecter ou à diagnostiquer.

Ce sont justement ces périodes de troubles du sommeil de type AER, pendant lesquelles le patient est soumis à des limitations de volume respiratoire moins importantes que celles ayant lieu durant une période d'apnée ou d'hypopnée, que la présente invention permet de détecter efficacement, de manière à permettre ensuite à un médecin, praticien ou analogue de déduire des données recueillies une information exploitable et fiable pour permettre l'établissement d'un diagnostic et la mise en oeuvre d'un traitement efficace du patient.

## Revendications

1. Procédé de fonctionnement d'un appareil de diagnostic ou de traitement des troubles respiratoires du sommeil d'un utilisateur, ledit appareil comprenant au moins une turbine motorisé permettant de délivrer un flux de gaz respiratoire sous pression variable ou constante dans au moins un circuit patient susceptible d'être relié audit utilisateur, comprenant les étapes de :
(a) détermination d'une image du débit respiratoire (IdD) du patient à partir d'une détection et/ou d'un suivi des variations du courant électrique consommé par la turbine pendant au moins une partie d'au moins un cycle respiratoire donné ;
(b) détermination d'une image du volume respiratoire (IdV) du patient par intégration de l'image du débit respiratoire du patient (IdD) pour obtenir l'aire (Acc) du courant consommé durant au moins au moins une partie dudit au moins un cycle respiratoire donné ;
(c) détermination de la moyenne mobile (mm-ldV) de l'image du volume respiratoire du patient sur les n cycles respiratoires ayant précédé ledit au moins un cycle respiratoire donné, avec n > 5 ;
(d) détermination d'au moins une information représentative d'au moins une limitation de débit respiratoire durant au moins ledit cycle respiratoire donné :
(i) par comparaison de l'image du volume respiratoire (IdV) déterminée à l'étape (b) avec la moyenne mobile (mm-ldV) de l'image du volume respiratoire déterminée à l'étape (c), et/ou
(ii) par calcul du rapport entre l'image du volume respiratoire (IdV) déterminée à l'étape (b) et l'aire (Asp) d'un sinus pur préfixé et/ou l'aire (Asc) d'un signal carré préfixé ;
(e) mémorisation et/ou affichage et/ou impression d'au moins une information déterminée à l'étape (d).

2. Procédé selon la revendication 1, caractérisé en ce qu'à l'étape (a), on détermine une image du débit respiratoire (IdD) et/ou à l'étape (b), on détermine une image du volume respiratoire (IdV) sur au moins une partie d'au moins 2 cycles respiratoires donnés successifs, de préférence sur 3 ou 4 cycles respiratoires donnés successifs.

3. Procédé selon l'une des revendications 1 ou 2, caractérisé en ce qu'à l'étape (c), la moyenne mobile (mm-ldV) de l'image du volume respiratoire du patient est déterminée sur les n cycles respiratoires ayant précédé ledit au moins un cycle respiratoire donné, avec n > 10, de préférence n est compris entre 15 et 40, préférentiellement encore entre 20 et 30.

4. Procédé selon l'une des revendications 1 à 3, caractérisé en ce qu'il comporte, en outre, au moins une étape de mémorisation et/ou affichage et/ou impression d'au moins une donnée choisie dans le groupe formé par :
- l'image du débit respiratoire (IdD),
- l'image du volume respiratoire (IdV), et
- la moyenne mobile (mm-ldV) de l'image du volume respiratoire.

5. Procédé selon l'une des revendications 1 à 4, caractérisé en ce qu'à l'étape (d), on soustrait une valeur de moyenne mobile (mm-ldV) de l'image du volume respiratoire déterminée à l'étape (c) à la valeur de l'aire (Acc) du courant consommé correspondant à l'image du volume respiratoire (IdV) déterminée à l'étape (b).

6. Appareil de diagnostic ou de traitement des troubles respiratoires du sommeil d'un utilisateur susceptible de fonctionner selon un procédé de fonctionnement selon l'une des revendications 1 à 5 , ledit appareil comprenant un circuit de gaz susceptible d'être relié audit utilisateur et au moins une turbine motorisé permettant de délivrer un flux de gaz respiratoire sous pression variable ou constante dans au moins ledit circuit de gaz, comprenant :
- des moyens de détection et/ou de suivi des variations du courant électrique consommé par la turbine pendant au moins une partie d'au moins un cycle respiratoire donné ;
- des moyens de détermination d'une image du débit respiratoire (IdD) du patient ;
- des moyens de détermination d'une image du volume respiratoire (IdV) du patient représentée par l'aire (Acc) du courant consommé durant au moins au moins une partie dudit au moins un cycle respiratoire donné, comprenant des moyens intégration de l'image du débit respiratoire du patient (IdD) pour obtenir ladite l'aire (Acc) du courant consommé ;
- des moyens de détermination de la moyenne mobile (mm-ldV) de l'image du volume respiratoire du patient sur les n cycles respiratoires ayant précédé ledit au moins un cycle respiratoire donné, avec n > 5 ;
- des moyens de détermination d'au moins une information représentative d'au moins une limitation de débit respiratoire durant au moins ledit cycle respiratoire donné (i) par comparaison de l'image du volume respiratoire (IdV) déterminée à l'étape (b) avec la moyenne mobile (mm-ldV) de l'image du volume respiratoire déterminée à l'étape (c), et/ou (ii) par calcul du rapport entre l'image du volume respiratoire (IdV) déterminée à l'étape (b) et l'aire (Asp) d'un sinus pur préfixé et/ou l'aire (Asc) d'un signal carré préfixé ; et
- des moyens de mémorisation et/ou affichage et/ou impression d'au moins une information de débit respiratoire et/ou d'au moins une donnée choisie dans le groupe formé par l'image du débit respiratoire (IdD), l'image du volume respiratoire (IdV), et la moyenne mobile (mm-ldV) de l'image du volume respiratoire.

7. Appareil selon la revendication 6, caractérisé en ce que les moyens de détection et/ou de suivi des variations du courant électrique consommé par la turbine permettent de déterminer une image du débit respiratoire (IdD) et/ou de déterminer une image du volume respiratoire (IdV) sur au moins une partie d'au moins 2 cycles respiratoires donnés successifs, de préférence sur 3 ou 4 cycles respiratoires donnés successifs.

8. Appareil selon l'une des revendications 6 ou 7, caractérisé en ce que les moyens de détermination de la moyenne mobile permettant de déterminer la moyenne mobile (mm-ldV) de l'image du volume respiratoire du patient sur n cycles respiratoires ayant précédé au moins un cycle respiratoire donné, avec n > 10, de préférence n est compris entre 15 et 40, préférentiellement encore entre 20 et 30.

9. Appareil selon l'une des revendications 6 à 8, caractérisé en ce que les moyens de détermination d'au moins une information représentative d'au moins une limitation de débit respiratoire permettant de soustraitre une valeur de moyenne mobile (mm-ldV) de l'image du volume respiratoire déterminée par les moyens de détermination de la moyenne mobile à la valeur de l'aire (Acc) du courant consommé correspondant à l'image du volume respiratoire (IdV) déterminée par les moyens de détermination d'une image du volume respiratoire (IdV).
